# EUROPEAN PATENT SPECIFICATION

(11) **EP 1 492 500 B1**
(45) Date of publication and mention of the grant of the patent: **16.07.2008**
(21) Application number: 03746589.5
(22) Date of filing: 01.04.2003
(51) Int. Cl.: A61K 9/12, A61K 9/72, A61K 31/58, A61K 31/167, A61K 47/10, A61K 47/26

(54) **FORMOTEROL AND CICLESONIDE AEROSOL FORMULATIONS**
FORMOTEROL UND CICLESONID AEROSOL-FORMULIERUNGEN
FORMULATIONS D'AEROSOL DE FORMOTEROL ET DE CICLESONIDE

(30) Priority: 05.04.2002 GB 0207899
(43) Date of publication of application: 05.01.2005
(73) Proprietor: 3M Innovative Properties Company, St. Paul, MN 55133-3427 (US)
(72) Inventor: OLIVER, Martin, J., Clarendon Park, Leicestershire LE2 1WS (GB); JINKS, Philip, A., Loughborough, Leicestershire LE11 3JS (GB)
(74) Representative: Aleandri-Hachgenei, Lorraine E.
(86) International application number: PCT/US2003/010285
(87) International publication number: WO 2003/086349

(56) References cited:
- WO-A-97/47286
- WO-A-98/52542
- WO-A-99/65460
- WO-A-99/65464
- DE-A- 19 541 689

## Description

### Field of the Invention

This invention relates to medicinal aerosol formulations and in particular to aerosol formulations containing formoterol in suspension and ciclesonide in solution suitable for administration to the respiratory tract.

### Background

Formoterol, N-[2-hydroxy-5-(1-hydroxy-2-((2-(4-methoxyphenyl)-1-methylethyl) amino)ethyl) phenyl] formamide, particularly in the form of its fumarate salt, is a bronchodilator used in the treatment of inflammatory or obstructive airways diseases.

GB-2247680 discloses pregna-1,4-diene-3,20-dione-16-17-acetal-21 esters and their use in the treatment of inflammatory conditions. The compounds have the general structure: 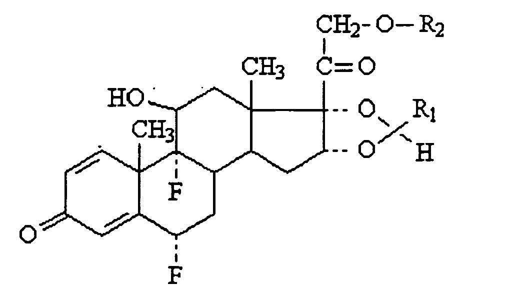 wherein R₁ is 2-propyl, 1-butyl, 2-butyl, cyclohexyl or phenyl; and R₂ is acetyl or isobutanoyl. Ciclesonide is 11β, 16α, 17, 21-tetrahydroxypregna 1,4-diene-3,20-dione, cyclic 16,17-acetal with cyclohexanecarboxaldehyde, 21-isobutyrate having the structure of general formula given above without fluorine atoms and in which R₁ is cyclohexyl and R₂ is isobutanoyl.

DE 19541689 discloses the combined use of ciclesonide with a β2-sympathomimetic, for the treatment of respiratory disorders. It is stated that such combinations are suitable for long-term therapy and provide good local and antiinflammatory effect in conjunction with quick relief of bronchospasms and without systemic side effects. As an exemplary formulation, DE 19541689 discloses a suspension aerosol composition consisting of ciclesonide (3.7 g) and formoterol fumarate dihydrate (1.1 g) as micronized particles dispersed in trichlorofluoromethane (1.99 kg), dichlorodifluoromethane (3.00 g) with sorbitan trioleate (15.5 g).

However at the time of the filing of DE 19541689 in 1995, these chlorofluorocarbon (CFC) propellants were generally understood to provoke the degradation of stratospheric ozone. Thus there was at that time and there still is a general need to provide aerosol formulations for medicaments, which employ so-called "ozone-friendly" propellants. A class of propellants which are believed to have minimal ozone-depleting effects in comparison to conventional CFCs comprise hydrofluorocarbons, in particular 1,1,1,2-tetrafluoroethane ("HFA 134a") and 1,1,1,2,3,3,3-heptafluoropropane ("HFA 227"). A number of medicinal aerosol formulations using such propellant systems are disclosed in, for example, EP 0372777, W091/04011, W091/11173, W091/11496, W091/14422, EP 0 504 112, W093/11745, WO 93/11747, WO 97/47286 and WO 98/52542. These applications are all concerned with the preparation of pressurised aerosols for the administration of medicaments and seek to overcome problems associated with the use of this new class of propellants, in particular the problems of stability associated with the pharmaceutical formulations prepared.

EP-A-0504112, for example, discloses a pharmaceutical composition for aerosol use containing: (a) a liquefied propellant gas or propellant gas mixture with a vapor pressure exceeding 1 bar but less than 6 bar (20°C) from the unsubstituted or partially to completely fluorinated hydrocarbon group; (b) a non-ionic tensile of the monoacetylated or diacetylated monoglyceride group;(c) a pharmaceutical active substance or combination of active substances, and, if necessary,(d) other common pharmaceutical accessory substances suitable for aerosol formulations. It is stated the basic purpose of that invention was to find a special accessory suspending substance for active substances in aerosol formulations, which dissolves better in liquefied "alternative" propellant gases than the accessory suspending substances hitherto recognized and used. It is stated that surprisingly, it was discovered, in solving this problem, that non-ionic tensides of the monoacetylated or diacetylated monoglyceride group are very soluble in the "alternative" propellant gases mentioned, particularly in HFA 227, are beneficial to the production of homogenous suspensions, and also have outstanding metering valve lubrication properties. Some of the examples of EP-A-0 504 112 disclose formulations comprising formoterol fumarate.

WO 93/11747 discloses a pharmaceutical suspension formulation suitable for aerosol administration, consisting essentially of a therapeutically effective amount of a drug and a propellant selected from the group consisting of HFA 134a, HFA 227, and a mixture thereof, the formulation being further characterized in that it exhibits substantially no growth in particle size or change in crystal morphology of the drug over a prolonged period, is substantially and readily redispersible, and upon redispersion does not flocculate so quickly as to prevent reproducible dosing of the drug. The application specifically discloses formulations of formoterol fumarate in HFA 134a, HFA 227 and 1:1 mixtures of HFA 134a and HFA 227. The formulations do not contain surfactants or ethanol. It is stated that mixtures of HFA 134a and HFA 227 may be adjusted for density matching with the drug.

WO 93/11745 discloses pharmaceutical aerosol formulations, substantially free of surfactant containing fluorocarbon or hydrogen-containing chlorofluorocarbon propellants and up to 5% of a polar co-solvent. Preferred propellants are HFA 134a and HFA 227 which are preferably used alone. The preferred polar co-solvent is ethanol and it is stated that in general only small quantities e.g. 0.05 to 3.0% w/w of polar co-solvent are required to improve the dispersion and the use of quantities in excess of 5% w/w may disadvantageously tend to dissolve the medicament.

WO 97/47286 discloses a pharmaceutical suspension formulation suitable for aerosol administration, consisting essentially of: (a) from 0.0025 to 0.1 % w/w of micronized formoterol, or an acid addition salt thereof and (b) from 0.1 to 5.0% w/w ethanol, (c) HFA 134a, HFA 227 or a mixture of HFA 227 and HFA 134a and optionally (d) a surfactant other than a monoacetylated or diacetylated monoglyceride, the formulation being further **characterized in that** it exhibits substantially no growth in particle size or change in crystal morphology of the drug over a prolonged period, is substantially and readily redispersible, and upon redispersion does not flocculate so quickly as to prevent reproducible dosing of the drug. The application specifically discloses formulations comprising formoterol fumarate dispersed in HFA 134a, HFA 227 or mixtures thereof and 1 to 3% ethanol. It is stated that it is important to ensure the formoterol fumarate does not come into contact with high concentrations e.g. above 10% w/w, of ethanol since the drug would dissolve leading to instability and crystal growth problems in the final formulation and that the maximum concentration of ethanol during formulation is preferably less than 5%. It is stated that aerosol compositions consisting of formoterol fumarate, HFA 134a and ethanol have proved to be extremely sensitive to ethanol concentration and an ethanol concentration of 3.5% w/w may cause unacceptable crystal growth.

WO 98/52542 discloses a pharmaceutical compositions comprising a therapeutically effective amount of a compound of the formula (I): 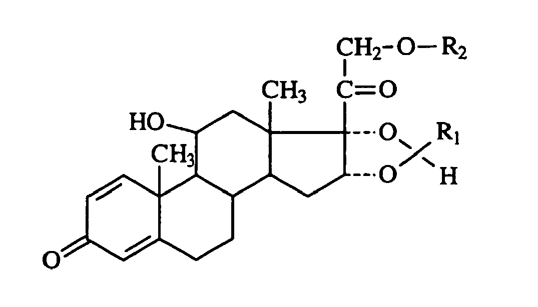 in which: R₁ is 1-butyl, 2-butyl, cyclohexyl or phenyl and R₂ is acetyl or isobutanoyl, and a hydrofluorocarbon propellant, preferably selected from 1,1,1,2-tetrafluoroethane, 1,1,1,2,3,3,3-heptafluoropropane and a mixture thereof, and cosolvent, preferably ethanol, in an amount effective to solubilize the compound of formula (I) and optionally a surfactant. The application specifically discloses solution formulations comprising ciclesonide (1 to 5 mg/ml) in HFA 134a, HFA 227 or mixtures of HFA 134a and HFA 227 and 5 to 20% by weight ethanol.

Despite the various approaches used in formulating drugs for use in aerosol inhalation, a number of serious difficulties and uncertainties are still often encountered in attempting to develop a physically and chemically stable CFC-free formulation that reliably delivers an accurate dose of drug having the proper particle size range.

### Summary of the Invention

There is a need for a CFC-free medicinal aerosol product containing formoterol and ciclesonide (or similar molecules) that is chemically and physically stable and that is suitable for delivery to the respiratory system of a patient.

Surprisingly it has been found that it is possible to provide physically and chemically stable formulations of formoterol fumarate in suspension and ciclesonide in solution at therapeutic effective concentrations in HFA 134a and/or HFA 227 propellant.

Accordingly in one aspect of the present invention there is provided a pharmaceutical aerosol composition comprising particles of formoterol or a pharmaceutically acceptable salt, solvate or physiologically functional derivative thereof, said particles being suspended in the formulation;
a compound of the formula (I): 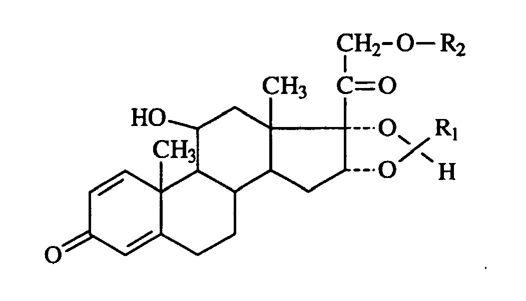 in which: R₁ is 1-butyl, 2-butyl, cyclohexyl or phenyl and R₂ is acetyl or isobutanoyl, said compound of formula (I) being dissolved in the formulation; and a propellant selected from 1,1,1,2-tetrafluoroethane, 1,1,1,2,3,3,3-heptafluoropropane and a mixture thereof.

WO 99/28296 and WO 99/65464 disclose medicinal aerosol formulations comprising a first drug suspended in propellant and a second drug dissolved in the formulation. However, ciclesonide or a similar molecule is not disclosed as a drug in either document, formoterol is not disclosed in WO 99/65644, and neither document discloses such a formulation containing formoterol or a pharmaceutically acceptable salt, solvate or physiologically functional derivative thereof and a compound of formula (I).

Due to the reported sensitivity of particulate formoterol, in particular formoterol fumarate, dispersed in HFA 134a and/or HFA 227 propellant to ethanol, it was considered not feasible to formulate particulate formoterol in combination with therapeutically effective amounts of ciclesonide (or similar molecules) at levels of ethanol that would be needed for complete dissolution of ciclesonide (or similar molecules).

Surprisingly, it was found possible to successfully formulate the drug combination at levels of ethanol considered unsuitable for one of the drugs. In particular, it was found that ethanol can be present in an amount effective to completely solubilize a compound of formula (I), such as ciclesonide, yet without the formoterol or a pharmaceutically acceptable salt, solvate or physiologically functional derivative thereof, such as formoterol fumarate, exhibiting detrimental growth in particle size or change in crystal morphology over a high stress storage interval.

The amount of ethanol in the formulation is advantageously present in a amount from 3 to 20% by weight, preferably from 3.5 to 12% by weight, more preferably from 3.5 to 10 % by weight, even more preferably from 5 to 10 % by weight, most preferably from 5 to 8% by weight.

Preferably the propellant comprises HFA 134a, more preferably HFA 134a is applied as the only propellant component.

To further enhance physical stability and homogeneity of the dispersion of formoterol or a pharmaceutically acceptable salt, solvate or physiologically functional derivative thereof, the formulations may advantageously comprise a particulate bulking agent having a mass median diameter of less than one micron.

Formulations according to the invention are particularly suitable for use in inhalation therapy, in which a therapeutically effective amount of the formulation is delivered to the lung by oral or nasal inhalation, more particularly for prophylaxis or treatment of a clinical condition for which a selective β₂-adrenoreceptor agonist and/or antiinflammatory corticosteroid is indicated. The present invention also provides a method for the prophylaxis or treatment of a clinical condition in a mammal, such as a human, for which a selective β₂-adrenoreceptor agonist and/or antiinflammatory corticosteroid is indicated, which comprises administration via inhalation a therapeutically effective amount of the formulation as described above. In particular, the present invention provides such methods for the prophylaxis or treatment of a disease associated with reversible airways obstruction such as asthma, chronic obstructive pulmonary disease (COPD), respiratory tract infection or upper respiratory tract disease.

In another aspect of the present invention, there is provided a dispenser comprising an aerosol vial equipped with a dispensing valve containing a formulation as described above.

### Detailed Description

It is to be understood that the present invention covers all combinations of particular and preferred aspects of the invention described herein.

As would be appreciated by the skilled person, formoterol includes two asymmetric centres. The present invention includes each isomer of formoterol either in substantially pure form or admixed in any proportions or a racemic mixture, particularly the (R, R)-isomer. The enantiomers of formoterol have been described previously, for example, in WO 98/21175 and US5795564.

By the term "physiologically functional derivative" is meant a chemical derivative of formoterol having the same physiological function as the free compound, for example, by being convertible in the body thereto. According to the present invention, examples of physiologically functional derivatives include esters.

Suitable salts according to the invention include those formed with both organic and inorganic acids. Pharmaceutical acceptable acid addition salts include but are not limited to those formed from hydrochloric, hydrobromic, sulphuric, citric, tartaric, phosphoric, lactic, pyruvic, acetic, trifluoroacetic, succinic, oxalic, fumaric, maleic, oxaloacetic, methanesulphonic, ethanesulphonic, p-toluenesulphonic, benzenesulphonic, isethionic, and naphthalenecarboxylic, such as 1-hydroxy-2-naphthalenecarboxylic acids.

Pharmaceutically acceptable esters of formoterol may have a hydroxyl group converted to a C₁₋₆ alkyl, aryl, aryl C ₁₋₆ alkyl, hetaryl (such as furanyl) or amino acid ester.

In preferred embodiments of the invention, formoterol fumarate (suitably as in the form of the dihydrate) is applied in combination with the compound of formula (I).

The compound of formula (I) is preferably ciclesonide.

Hereinafter, the term "formoterol " is understood to include formoterol or a pharmaceutical acceptable salt, solvate, or physiologically functional derivative thereof, preferably formoterol fumarate, more preferably formoterol fumarate dihydrate, while the term "compound of formula (I)" includes preferably ciclesonide.

Formoterol and the compound of formula (I) are generally present in a formulation of the invention in a therapeutically effective amount.

The amount of formoterol and compound of formula (I), which is required to achieve a therapeutic effect will, of course, vary with the particular compound, the subject under treatment, and the particular disorder or disease being treated. Suitably, the pharmaceutical formulations which are suitable for inhalation according to the invention comprise formoterol and a compound of formula (I) in amounts such that one or two actuations provide a therapeutically effective dose, for example, a dose of formoterol of 1 mcg to 50 mcg, preferably 3 mcg to 25 mcg, more preferably 4 mcg to 12 mcg and a dose of the compound of Formula (I) of 20 mcg to 1 mg, preferably 35 mcg to 500 mcg, more preferably, 50 mcg to 200 mcg. Various dosing of the individual drugs can be advantageously combined for particular disorders or subjects under treatment.

Preferably formoterol constitutes about 0.06 to about 0.60 mg per ml, more preferably about 0.08 to about 0.30 mg per ml, most preferably about 0.10 to about 0.20 mg per ml of the formulation.

The particles of formoterol are generally micronised particles or particles processed by other methods, preferably having a mass median diameter equal to or greater than 1 micron, more particularly from 1 to 10 micron, even more particularly from 1 to 5 micron. Smaller particles having a mass median diameter of less than one micron may also be suitable.

The compound of formula (I) is generally present at a concentration of from about 0.5 to about 8 mg per ml, preferably about 1 to about 5 mg per ml, most preferably about 1 to about 4 mg per ml of the formulation.

The formulations of the invention typically comprise an adjuvant to aid the complete dissolution of the compound of formula (I). The level of adjuvant is desirably selected such that the compound of formula (I) is completely soluble in the aerosol formulation over the temperature range likely to be encountered by the product during use, e.g. 10 to 35°C, while the suspended formoterol exhibits substantially no growth in particle size (e.g. a growth of 25% or less (e.g. based on mass median diameter as determined by laser diffraction) after high stress cycling (i.e. 4 temperature cycles in a 24 hour period, where one cycle is defined as: 4°C at a duration of 2 h; ramping up from 4 to 40°C in 1 h; 40°C for 2 h and ramping down from 40 to 4°C in 1 h) over a period of 10 days). Suitable adjuvants are disclosed in EP-A-0372777. Ethanol is a preferred adjuvant. When ethanol is applied as adjuvant, it has been found that ethanol is desirably present in an amount from 3 to 20% by weight, preferably from 3.5 to 12% by weight, more preferably from 3.5 to 10 % by weight, even more preferably from 5 to 10 %, most preferably from 5 to 8% by weight of the formulation.

The propellant is selected from HFA 134a, HFA 227 and mixtures thereof. Preferably the propellant comprises HFA 134a, either in a mixture with HFA 227 or more preferably as the only propellant component. Variation of the concentration of HFA 134a and HFA 227 in mixtures allows adjustment of the density of the propellant to match the density of the suspended formoterol. Density matching may decrease the rate of sedimentation or creaming of the suspended formoterol particles.

For formulations comprising ethanol as adjuvant and HFA 134a and/or HFA 227 as propellant, it has been observed that the desired level of ethanol may vary in relation to the particular selection of propellant. Surprisingly, such formulations including HFA 134a as the only propellant demonstrate superior performance "over HFA 227 only formulations", e.g. enhanced stability of suspended formoterol particles, although such formulations typically require higher levels of ethanol for the complete dissolution of the compound of formula (I) e.g. over a temperature range of 10 to 35 °C (in comparison to formulations with HFA 227 as the only propellant). Further, it has been surprisingly found that for formulations with HFA 134a as the only propellant component and containing concentrations of the compound of formula (I), such that a level of ethanol of less than 5 % by weight may be sufficient to completely dissolve said compound e.g. over a temperature range of 10 to 35°C, the application of higher levels of ethanol (5 % or more) is advantageous, in that such formulations show superior uniformity in through life content testing and/or superior results in loss in dose testing for both the compound of formula (I) and formoterol.

The aerosol formulations of the invention may preferably contain surfactant, more preferably a surfactant other than a monoacetylated or diacetylated monoglyceride, for e.g. imparting a flocculant effect for the suspended formoterol, which may allow less migration of the drug to and from the metering chamber. When surfactant is included in a formulation it is generally present in an amount of about 0.001 % to 0.010 % by weight of the formulation. Suitable surfactants are well known in the art and include sorbitan trioleate, oleic acid and lecithin. Surfactants, such as oligolactic acid derivatives disclosed in WO94/21228 and WO94/21229, and other surfactants disclosed in the literature may be used. As a surfactant oleic acid is preferred. The formulations are preferably free of other excipients.

It has been found that a bulking agent having a mass median diameter of less than one micron may be applied to enhance physical stability and homogeneity of the suspension of the formoterol particles. It is not necessary for the surface of the bulking agent or the drug to be coated with a surface modifier to achieve improved stability. In particular it has been found that the application of such a nano-sized bulking agent aids in minimizing the tendency of formoterol to cream or sediment, depending on the density difference of the drug and the propellant. More particularly, it has been found that the application of such a nano-sized bulking agent aids in maintaining a high sediment volume (i.e. minimizing a dense packing of the sediment) and/or the formation of a voluminous, loosely flocculated matrix, enhancing the re-dispersion and/or de-flocculation of the drug upon agitation.

The mass median diameter of the bulking agent can advantageously be as low as 300 nanometers, more desirably as low as 250 nanometers, even more desirably the mass median diameter is in the range of 100 to 250 nanometers and most desirably in the range of 150 to 200 nanometers.

Mass median diameter (which is equivalent to volume median diameter) can be determined using any conventional particle size measurement method known to those skilled in the art. Suitable methods include for example laser diffraction, photon correlation spectroscopy (e.g. using a spectrometer available under the trade designation Brookhaven PCS from Brookhaven Inc.), spinning disc centrifuging (using an instrument available under the trade designation CPS Disc Centrifuge from Chemical Process Specialists Inc.) and scanning electron microscopy (SEM). Mass median diameter is preferably determined by laser diffraction, photon correlation spectroscopy or spinning disc centrifuging, more preferably by laser diffraction, more particularly laser diffraction using an analyser available under the trade designation Malvern Mastersizer 2000 laser light diffraction particle size analyser from Malvern Instruments Ltd.

Preferred bulking agents include lactose, DL-alanine, ascorbic acid, glucose, sucrose D(+)trehalose as well as their various hydrates, anomers and/or enantiomers. Lactose including its various forms, such as α-lactose monohydrate and β-lactose and alanine are more preferred. Lactose, in particular in its α-lactose monohydrate form, is most preferred as a bulking agent due to e.g. processing considerations. Other suitable bulking agents include other saccharides e.g. D-galactose, maltose, D(+)raffinose pentahydrate, sodium saccharin, polysaccharides e.g. starches, modified celluloses, dextrins or dextrans, other amino acids e.g. glycine, salts e.g. sodium chloride, calcium carbonate, sodium tartrate, calcium lactate, or other organic compounds e.g. urea or propyliodone.

Based on the amount of formoterol in the formulation, the weight ratio of formoterol to bulking agent is generally in the range 1:0.1 to 1:25, preferably 1:2 to 1:15, even more preferably 1:4 to1:12 and most preferably about 1:10.

The bulking agent may be reduced to the required particle size by any convenient method, e.g. grinding, air-jet milling etc. Preferably the bulking agent is reduced to nanoparticle size in a high pressure homogenizer, such as the commercially available Avestin Emulsiflex homogenizers and the Microfluidics Microfluidizer homogenizers. In the processing with high pressure homogenizers, certain bulking agents can be reduced to the desired particle size using lower pressures than that applied for other bulking agents. For example, it has been found that lactose, more specifically α-lactose monohydrate, can be effectively reduced to the desired particle size using pressures between about 10,000 and about 21,000 psi, while for effective particle size reduction of alanine or sucrose higher pressures of about 25,000 psi for repeated passes were applied.

The bulking agent may be prepared in a slurrying aid which is a low volatility solvent such as ethanol. It may be prepared in a slurrying aid which is a component of the final aerosol formulation, or it may be prepared in a solvent that is subsequently removed or exchanged with a component of the formulation by some process such as centrifugation and decanting, dialysis, evaporation etc.

It is particularly convenient to use a slurrying aid in the high pressure homogenizer which is a low volatility component of the aerosol formulation and after particle size reduction has been achieved the slurry may be adjusted if necessary, e.g. concentrated by centrifugation, decanting etc. Whilst it has been found that slurries with excessively high powder loadings may be difficult to process due to their rheological properties, it is generally advantageous to process slurries with powder loading concentrations which approach this processing limit in order to achieve the desired particle size distribution in the shortest processing time. Thus, the weight ratio of liquid:solid is generally in the range 5:1 to 100:1, preferably 5:1 to 20:1, and most preferably about 8:1 to about 10:1.

The present invention also provides a method of preparing a formulation according to the invention, the method comprising the steps of (i) providing a solution of the compound of formula (I) in HFA 134a and/or HFA 227 and (ii) dispersing particles of formoterol in the solution. For formulations containing adjuvant, in particular ethanol, typically step (i) includes sub-steps of mixing the compound of formula (I), and if applicable surfactant, in an appropriate amount of adjuvant and adding the resultant to an appropriate amount of HFA 134a and/or HFA 227 in liquid form (chilled to below its boiling point or range). Step (ii) typically includes the following sub-steps: removing a portion of the compound of formula (I) containing solution, adding particulate formoterol to this portion to form a formoterol-containing slurry, mixing the formoterol-containing slurry, preferably after high shear mixing thereof, in the remaining portion of the original compound of formula (I) containing solution.

For formulations containing a nano-sized bulking agent, a preferred method of preparing a formulation comprises the steps of (i) forming a slurry of bulking agent with a component of the formulation; (ii) subjecting the slurry to high pressure homogenization; and (iii) combining the resulting slurry with other components of the aerosol formulation. For formulations containing ethanol, the slurry of bulking agent may be advantageously prepared with an appropriate amount of ethanol. The slurry is subjected to high pressure homogenization prior to adding it to the remainder of the formulation. During manufacture, typically the slurry of bulking agent is then added to a solution of the compound of formula (I) in an appropriate amount of HFA 134a and/or HFA 227 and, if applicable an appropriate amount of adjuvant (e.g. ethanol) and/or surfactant. (Said solution prepared in a similar manner as described above.) In a subsequent step, particles of formoterol are then dispersed in compound of formula (I) containing liquid. As described above, this is typically achieved by taking off a portion of the compound of formula (I) containing liquid, in an intermediate step, and adding particulate formoterol to this portion to form a slurry of formoterol. This formoterol slurry, typically after high shear mixing thereof, is then re-added and mixed with the remaining portion of the original compound of formula (I) mixture.

Dispensers comprising an aerosol vial equipped with conventional dispensing valves, preferably metered dose valves, can be used to deliver formulations of the invention. Conventional dispensers and aerosol vials can be used to contain a formulation of the invention. However certain vials may enhance the chemical stability of certain formulations of the invention. Therefore it is preferred to contain a formulation of the invention within a glass aerosol vial or a metal, in particular aluminum, vial having an interior surface coated with a polymer, in particular a fluorocarbon polymer. Advantageously other internal surfaces, in particular such surfaces of components of the valve, or all of the internal surfaces of the dispenser may be also coated with a coating comprising a polymer, in particular a fluorocarbon polymer. Suitable fluorocarbon polymers include fluorocarbon polymers, which are made of multiples of one or more of the following monomeric units: tetrafluoroethylene (PTFE), fluorinated ethylene propylene (FEP), perfluoroalkoxyalkane (PFA), ethylene tetrafluoroethylene (ETFE), vinylidenefluoride (PVDF), and chlorinated ethylene tetrafluoroethylene. Polymers, which have a relatively high ratio of fluorine to carbon, such as perfluorocarbon polymers e.g. PTFE, PFA, and FEP, are preferred; FEP is particularly preferred.

The valve may be any suitable metering valve with an outlet made from, for example stainless steel, acetal, nylon or polybutylene terephthalate and with seals made from nitrile or EPDM elastomer.

A formulation of the invention can be administered to the lung by oral or nasal inhalation. Oral inhalation is preferred, and conventional actuators for oral inhalation can be used in connection with a formulation of the invention. Good respirable doses can be achieved with an orifice diameter within the range of 0.2 to 0.6 mm, preferably in the range 0.24 to 0.47 mm, most preferably 0.28 to 0.35 mm.

The invention will be illustrated by the following Examples.

### Materials used:

α-lactose monohydrate supplied under the trade designation Pharmatose 325M by DMV International Pharma was micronised by fluid energy milling in a single pass (referred to here and in the following as "micronised lactose monohydrate"). Micronised lactose monohydrate (100 g) was dispersed in Anhydrous Ethanol (840 g) using a Silverson high shear mixer. This dispersion was added to the product reservoir of an Avestin Emulsiflex C50 homogenizer, and recirculated for 20 minutes at 10,000 psi. The dispersion was then passed out of the homogenizer, after recirculation for 30 seconds, at 20,000 psi. The particle size was determined according to the following method.

### Particle Size Analysis

For analysis of a Lactose/Ethanol slurry, a (0.5 ml) sample of the slurry, which was shaken for at least one minute to ensure homogeneity, was added to a solution of 0.05% Lecithin in Iso-octane (20 ml), and redispersed with mild ultrasonics for 1 minute.

The resulting suspension was introduced dropwise into the presentation cell (a Hydro 2000 SM small sample presentation cell) of a Malvern Mastersizer 2000^{™} laser diffraction particle sizer until the obscuration was in the working range (between 10 and 12 with a red laser), and left to circulate for 1 minute to allow complete mixing and thermal equilibrium to be established. Ten readings were taken at 20 second intervals to establish that the particle size was stable. The General Purpose analysis model, as described in the Malvern Instruments Operators Guide, was used with refractive indices 1.533 (lactose), 1.392 (iso-octane) and absorbance 0.001 (lactose). The results are based on the average calculated results of 10 readings taken in succession. The procedure was performed twice.

### Results of Particle Size Analysis by Malvern Mastersizer 2000

| | Lactose |
|---|---|
| Units | Microns |
| | |
| d(v,0.1) | 0.073 |
| d(v,0.5) median | 0.170 |
| d(v,0.9) | 1.259 |
| D[4,3] volume | 0.455 |
| weighted mean | |

| | |
|---|---|
| Units | Percent |
| vol under 0.05 micron | 2.10 |
| vol under 0.10 micron | 23.10 |
| vol under 0.20 micron | 57.48 |
| vol under 0.50 micron | 76.49 |
| vol under 1.0 micron | 86.56 |
| vol under 2.0 micron | 95.97 |
| vol under 5.0 micron | 99.54 |
| vol under 10.0 micron | 100.00 |
| vol under 20.0 micron | 100.00 |

### Examples

### Example 1

| **Material** | **mg/ml** | **% w/w** | **Batch quantity (g)** |
|---|---|---|---|
| FORMOTEROL FUMARATE dihydrate (micronised) | 0.120 | 0.0101 | 0.1140 |
| CICLESONIDE (micronised) | 4.000 | 0.3362 | 3.7989 |
| OLEIC ACID (VEGETABLE SOURCE) Ph.Eur/USNF | 0.0595 | 0.0050 | 0.0565 |
| Dehydrated Alcohol USP; Ethanol, Anhydrous Ph. Eur. | 59.4913 | 5.0000 | 56.5000 |
| PROPELLANT 134a, | 1126.1561 | 94.6487 | 1069.5306 |
| Total | 1189.8269 | 100.0000 | 1130.0000 |

This formulation was prepared as follows. HFA 134a was weighed into a batching vessel and stored at -60°C. Oleic acid and Ciclesonide were dissolved in Ethanol and the solution added to the batching vessel to produce a chilled blend. The formoterol fumarate was then dispersed in the chilled blend using a high shear mixer. The resulting chilled suspension was filled into 10ml aluminium vials whose internal surface was lined with Fluorinated Ethylene Propylene (FEP) polymeric coating. The vials were immediately sealed with 50 mcl metering valves. A fill weight of 11.3 g was used and 100 MDI units were prepared.

### Example 2

| **Material** | **mg/ml** | **% w/w** | **Batch quantity (g)** |
|---|---|---|---|
| FORMOTEROL FUMARATE dihydrate (micronised) | 0.1200 | 0.0101 | 0.1140 |
| CICLESONIDE (micronised) | 4.0000 | 0.3362 | 3.7989 |
| LACTOSE MONOHYDRATE Ph. Eur./USNF | 1.2000 | 0.1009 | 1.1397 |
| OLEIC ACID (VEGETABLE SOURCE) Ph.Eur/USNF | 0.0595 | 0.0050 | 0.0565 |
| Dehydrated Alcohol USP; Ethanol, Anhydrous Ph. Eur. | 59.4913 | 5.0000 | 56.5000 |
| PROPELLANT 134a, | 1124.9561 | 94.5478 | 1068.3909 |
| Total | 1189.8269 | 100.0000 | 1130.0000 |

This formulation was prepared as follows. HFA 134a was weighed into a batching vessel and stored at -60°C. Oleic acid and Ciclesonide were dissolved in 46.9265 g of the Ethanol. Nano-sized lactose/ethanol slurry (10.7132 g) as prepared above was added to the solution of oleic acid and Ciclesonide in ethanol, and this mixture added to the HFA 134a in the batching vessel at -60°C to produce a chilled blend, The formoterol fumarate was then dispersed in the chilled blend using a high shear mixer. The resulting chilled suspension was filled into 10ml FEP-lined aluminium vials, which were immediately sealed with 50 mcl metering valves. A fill weight of 11.3 g was used and 100 MDI units were prepared.

### Evaluation of Formulations

The suspension settling rates of the formulations of Examples 1 and 2 were compared after cold-transferring formulation from 5 MDI units (of each example) to 5 clear plastic PET vials by chilling the units to -60°C, de-crimping the valves, pouring the contents into the PET vials and crimping on non-metering valves. After allowing the formulations in the clear vials to equilibrate to room temperature, they were compared by visual assessment. Here the physical characteristics were observed after the formulations were shaken and then left to stand. The formulation of Example 1 was seen to be homogeneous over a 10 minute standing period whereas the formulation of Example 2 sedimented during this period to form a sediment volume occupying approximately 30% of the total formulation volume. The formulations were re-examined after 18 hours standing and it was noted that the formulation of Example 1 had sedimented to form a sediment which occupied a volume of around 1% of the formulation volume, whereas the formulation of Example 2 still had a sediment volume occupying approximately 30% of the total formulation volume.

The above observations indicate the nano-sized lactose bulked formulation of Example 2 has far greater homogeneity after 18 hours standing than the corresponding non-bulked formulation of Example 1. The improved homogeneity of the formulation of Example 2 provides greater uniformity of dosing in an MDI system.

### Stability on Storage

MDI units from both Examples 1 and 2 were stored (in a valve up orientation) for 10 days at the following conditions: a) at temperature of 5°C; b) at temperature of 40°C and 75% relative humidity; and c) under temperature cycling between 4°C and 40°C with 4 temperature cycles in a 24 hour period (one cycle having the following phases: 4°C at a duration of 2 h; ramping up from 4 to 40°C in 1 h; 40°C for 2 h and ramping down from 40 to 4°C in 1 h).

For each Example, the contents of the three units from each storage condition were transferred to PET vials (as described above) and after allowing the formulation to equilibrate to room temperature examined visually (as described above). It was noted that there were no differences discernible in any of the formulations between any of the storage conditions.

The FEP-lined aluminium vials and valves were also examined and it was noted that there were no signs of crystal growth on the surfaces of the vial or valve and that there were no excessive levels of drug deposition.

Samples of Example 1 (transferred to PET vials) prior to storage and after storage at each condition were also evaluated for turbidity by using an optical measuring technique described in the Proceedings of Drug Delivery to the Lung VI p.10-13 (December 1995) printed by the Aerosol Society. To use the optical technique, each vial was shaken vigorously for 20 seconds before insertion into the apparatus, and light transmission through the formulation was measured after 30 seconds. The measurements were achieved by reading off the voltage value obtained from the light receiving sensor set to a central position relative to the initial height of the suspension column. A voltage 12.13 V corresponded to complete transmission, while 0 V corresponded to complete opacity.

The turbidity results were as follows:

| Light transmission reading (Volts) | Initial | 5°C | 40°C / 75% rh | Temperature cycling 4-40°C |
|---|---|---|---|---|
| Vial 1 | 2.78 | 3.09 | 2.72 | 2.98 |
| Vial 2 | 2.92 | 2.97 | 3.04 | 2.86 |
| Vial 3 | 2.90 | - | 2.74 | 3.02 |

The results indicate that the suspension showed no significant changes in turbidity indicating that formulations with combined active ingredients had a satisfactory level of particle size stability for the suspended formoterol fumarate.

### Chemical Stability

MDI units from Example 2 were stored for 5 days at 50°C in both valve-up and valve-down orientations (two units at each orientation). The samples were then tested for ciclesonide related impurities by High Performance Liquid Chromatography and the following results were obtained:

| Sample | % Impurity |
|---|---|
| 1 (valve-up) | 0.06 |
| 2 (valve-up) | 0.06 |
| 1 (valve-down) | 0.05 |
| 2 (valve-down) | 0.05 |

The results indicate that ciclesonide has a high degree of chemical stability in the example formulation; the value of 0.06% being just above the limit of quantification. Extrapolation of these results would suggest that this MDI system would have satisfactory long term (e.g. 2 to 3 years) chemical stability at 30°C.

## Claims

1. A pharmaceutical aerosol formulation comprising
particles of formoterol or a pharmaceutically acceptable salt, solvate or physiologically functional derivative thereof, said particles being suspended in the formulation;
a compound of the formula (I): 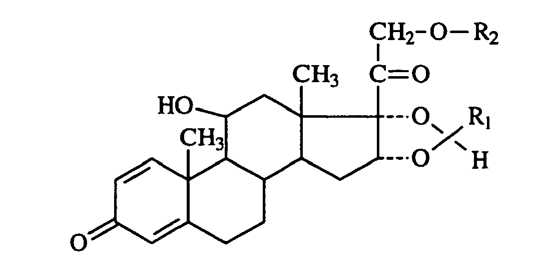 in which: R₁ is 1-butyl, 2-butyl, cyclohexyl or phenyl and R₂ is acetyl or isobutanoyl, said compound of formula (I) being dissolved in the formulation; and
a propellant selected from 1,1,1,2-tetrafluoroethane, 1,1,1,2,3,3,3-heptafluoropropane and a mixture thereof.

2. A pharmaceutical aerosol formulation according to claim 1, wherein the formoterol is in the form of fomoterol fumarate.

3. A pharmaceutical aerosol formulation according to claim 1 or claim 2, wherein the compound of formula (I) is ciclesonide.

4. A pharmaceutical aerosol formulation according to any one of claims 1 to 3, wherein the formulation further comprises ethanol.

5. A pharmaceutical aerosol formulation according to claim 4, wherein ethanol is present in an amount from 3 to 20% by weight of the formulation.

6. A pharmaceutical aerosol formulation according to claim 5, wherein ethanol is present in an amount from 3.5 to 20% by weight of the formulation.

7. A pharmaceutical aerosol formulation according to claim 6, wherein ethanol is present in an amount from 5 to 20% by weight of the formulation.

8. A pharmaceutical aerosol formulation according to any one of the claims 1 to 7, wherein the propellant is 1,1,1,2-tetrafluoroethane or a mixture of 1,1,1,2-tetrafluoroethane and 1,1,1,2,3,3,3-heptafluoropropane.

9. A pharmaceutical aerosol formulation according to any one of the claims 1 to 8, wherein 1,1,1,2-tetrafluoroethane is the only propellant component.

10. A pharmaceutical aerosol formulation according to any one of claims 1 to 9, wherein the formulation further comprises a bulking agent having a mass median diameter of less than one micron.

11. A pharmaceutical aerosol formulation according to claim 10, wherein the bulking agent has a mass median diameter of not more than 300 nm.

12. A pharmaceutical aerosol formulation according to claim 10 or claim 11, wherein the weight ratio of formoterol or a pharmaceutically acceptable salt, solvate or physiologically functional derivative thereof to bulking agent is in the range 1:0.1 to 1:25.

13. A dispenser comprising an aerosol vial equipped with a dispensing valve, said aerosol vial containing a formulation according to any one of claims 1 to 12.

14. A dispenser according to claim 13, wherein an interior surface of the aerosol vial is coated with a coating comprising a fluorocarbon polymer.

15. A method of preparing a formulation according to claim 1, the method comprising the steps of (i) providing a solution of the compound of formula (I) in 1,1,1,2-tetrafluoroethane and/or 1,1,1,2,3,3,3-heptafluoropropane and (ii) dispersing particles of formoterol or a pharmaceutically acceptable salt, solvate or physiologically functional derivative thereof in said solution.

## Patentansprüche

1. Pharmazeutische Aerosolrezeptur umfassend:
Teilchen von Formoterol oder eines pharmazeutisch akzeptablen Salzes, Solvats oder physiologisch funktionellen Derivats desselben, wobei die Teilchen in der Rezeptur suspendiert sind;
eine Verbindung der Formel (I): 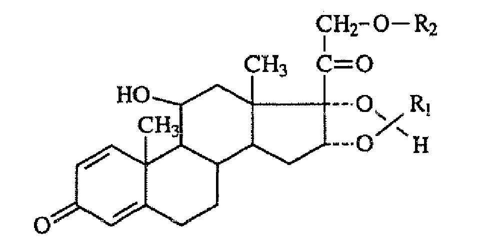
wobei: R₁ 1-Butyl, 2-Butyl, Cyclohexyl oder Phenyl ist und R₂ Acetyl oder Isobutanoyl ist, wobei die Verbindung der Formel (I) in der Rezeptur gelöst wird;
und
ein Treibmittel ausgewählt unter 1,1,1,2-Tetrafluorethan, 1,1,1,2,3,3,3-Heptafluorpropan und einer Mischung derselben.

2. Pharmazeutische Aerosolrezeptur nach Anspruch 1, wobei das Formoterol in Form von Formoterolfumarat vorliegt.

3. Pharmazeutische Aerosolrezeptur nach Anspruch 1 oder Anspruch 2, wobei die Verbindung der Formel (I) Ciclesonid ist.

4. Pharmazeutische Aerosolrezeptur nach einem der Ansprüche 1 bis 3, wobei die Rezeptur ferner Ethanol umfasst.

5. Pharmazeutische Aerosolrezeptur nach Anspruch 4, wobei Ethanol in einer Menge von 3 bis 20 Gew.-%, auf die Rezeptur bezogen, vorliegt.

6. Pharmazeutische Aerosolrezeptur nach Anspruch 5, wobei Ethanol in einer Menge von 3,5 bis 20 Gew.-%, auf die Rezeptur bezogen, vorliegt.

7. Pharmazeutische Aerosolrezeptur nach Anspruch 6, wobei Ethanol in einer Menge von 5 bis 20 Gew.-%, auf die Rezeptur bezogen, vorliegt.

8. Pharmazeutische Aerosolrezeptur nach einem der Ansprüche 1 bis 7, wobei das Treibmittel 1,1,1,2-Tetrafluorethan oder eine Mischung von 1,1,1,2-Tetrafluorethan und 1,1,1,2,3,3,3-Heptafluorpropan ist.

9. Pharmazeutische Aerosolrezeptur nach einem der Ansprüche 1 bis 8, wobei 1,1,1,2-Tetrafluorethan die einzige Treibmittelkomponente ist.

10. Pharmazeutische Aerosolrezeptur nach einem der Ansprüche 1 bis 9, wobei die Rezeptur ferner ein Füllmaterial umfasst, das einen mittleren Massendurchmesser von weniger als einem Mikron aufweist.

11. Pharmazeutische Aerosolrezeptur nach Anspruch 10, wobei das Füllmaterial einen mittleren Massendurchmesser von nicht mehr als 300 nm aufweist.

12. Pharmazeutische Aerosolrezeptur nach Anspruch 10 oder Anspruch 11, wobei das Gewichtsverhältnis von Formoterol oder eines pharmazeutisch akzeptablen Salzes, Solvats oder physiologisch funktionellen Derivats desselben zum Füllmaterial im Bereich von 1:0,1 bis 1:25 liegt.

13. Spendevorrichtung umfassend eine Aerosolphiole, die mit einem Spendeventil ausgestattet ist, wobei die Aerosolphiole eine Rezeptur nach einem der Ansprüche 1 bis 12 enthält.

14. Spendevorrichtung nach Anspruch 13, wobei eine Innenfläche der Aerosolphiole mit einer Beschichtung beschichtet ist, die ein Fluorkohlenstoffpolymer umfasst.

15. Verfahren für die Herstellung einer Rezeptur nach Anspruch 1, wobei das Verfahren die Schritte des (i) Bereitstellens einer Lösung der Verbindung der Formel (I) in 1,1,1,2-Tetrafluorethan und/oder 1,1,1,2,3,3,3-Heptafluorpropan und (ii) des Dispergierens von Teilchen von Formoterol oder eines pharmazeutisch akzeptablen Salzes, Solvats oder physiologisch funktionellen Derivats desselben in der Lösung umfasst.

## Revendications

1. Formulation pharmaceutique pour aérosol, comprenant
des particules de formotérol ou d'un sel, d'un solvate ou d'un dérivé physiologiquement fonctionnel, pharmaceutiquement acceptable de celui-ci, lesdites particules étant en suspension dans la formulation ;
un composé de formule (I) : 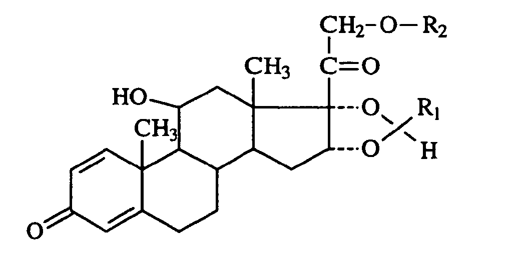 dans laquelle
R₁ représente 1-butyle, 2-butyle, cyclohexyle ou phényle et
R₂ représente acétyle ou isobutanoyle, ledit composé de formule (I) étant dissous dans la formulation ; et
un agent propulseur choisi parmi le 1,1,1,2-tétrafluoroéthane, le 1,1,1,2,3,3,3-heptafluoropropane et un mélange de ceux-ci.

2. Formulation pharmaceutique pour aérosol selon la revendication 1, où le formotérol est sous forme de fumarate de formotérol.

3. Formulation pharmaceutique pour aérosol selon la revendication 1 ou la revendication 2, où le composé de formule (I) est le ciclesonide.

4. Formulation pharmaceutique pour aérosol selon l'une quelconque des revendications 1 à 3, où la formulation comprend en outre de l'éthanol.

5. Formulation pharmaceutique pour aérosol selon la revendication 4 où l'éthanol est présent en une quantité de 3 à 20% en poids de la formulation.

6. Formulation pharmaceutique pour aérosol selon la revendication 5 où l'éthanol est présent en une quantité de 3,5 à 20% en poids de la formulation.

7. Formulation pharmaceutique pour aérosol selon la revendication 6 où l'éthanol est présent en une quantité de 5 à 20% en poids de la formulation.

8. Formulation pharmaceutique pour aérosol selon l'une quelconque des revendications 1 à 7, où l'agent propulseur est le 1,1,1,2-tétrafluoroéthane ou un mélange de 1,1,1,2-tétrafluoroéthane et de 1,1,1,2,3,3,3-heptafluoropropane.

9. Formulation pharmaceutique pour aérosol selon l'une quelconque des revendications 1 à 8, où le 1,1,1,2-tétrafluoroéthane est le seul composant propulseur.

10. Formulation pharmaceutique pour aérosol selon l'une quelconque des revendications 1 à 9, où la formulation comprend en outre un agent diluant présentant un diamètre massique moyen inférieur à un micron.

11. Formulation pharmaceutique pour aérosol selon la revendication 10, où l'agent diluant présente un diamètre massique moyen qui n'est pas supérieur à 300 nm.

12. Formulation pharmaceutique pour aérosol selon la revendication 10 ou la revendication 11, où le rapport pondéral du formotérol ou d'un sel, d'un solvate ou d'un dérivé physiologiquement fonctionnel, pharmaceutiquement acceptable de celui-ci à l'agent diluant est situé dans la plage de 1:0,1 à 1:25.

13. Distributeur comprenant une fiole à aérosol équipée d'une soupape de distribution, ladite fiole à aérosol contenant une formulation selon l'une quelconque des revendications 1 à 12.

14. Distributeur selon la revendication 13, où la surface intérieure de la fiole à aérosol est revêtue par un revêtement comprenant un polymère fluorocarboné.

15. Procédé de préparation d'une formulation selon la revendication 1, le procédé comprenant les étapes (i) de fourniture d'une solution du composé de formule (I) dans du 1,1,1,2-tétrafluoroéthane et/ou du 1,1,1,2,3,3,3-heptafluoropropane et (ii) de dispersion de particules de formotérol ou d'un sel, d'un solvate ou d'un dérivé physiologiquement fonctionnel de celui-ci, pharmaceutiquement acceptable, dans ladite solution.
